# EUROPEAN PATENT SPECIFICATION

(11) **EP 4 125 850 B1**
(45) Date of publication and mention of the grant of the patent: **20.11.2024**
(21) Application number: 21717498.6
(22) Date of filing: 31.03.2021
(51) Int. Cl.: A61K 31/19, A61K 31/593, A61K 31/592, A61P 31/16

(54) **SODIUM BUTYRATE FOR USE IN THE PREVENTION OR TREATMENT OF RHINOVIRUS INFECTION**
NATRIUMBUTYRAT ZUR VERWENDUNG BEI DER VORBEUGUNG ODER BEHANDLUNG VON RHINOVIRUSINFEKTIONEN
BUTYRATE DE SODIUM À UTILISER DANS LA PRÉVENTION OU LE TRAITEMENT D'UNE INFECTION À RHINOVIRUS

(30) Priority: 31.03.2020 GB 202004690
(43) Date of publication of application: 08.02.2023
(73) Proprietor: The Court of Edinburgh Napier University, Edinburgh EH14 1DJ (GB)
(72) Inventor: STEVENS, Craig, Edinburgh EH11 4BN (GB); BARLOW, Peter, Edinburgh EH11 4BN (GB)
(74) Representative: Jacob, Reuben Ellis
(86) International application number: PCT/GB2021/050809
(87) International publication number: WO 2021/198691

(56) References cited:
- WO-A1-2020/060426
- CN-A- 104 490 854
- US-A1- 2015 258 047
- US-A1- 2019 091 221
- THIO CHRISTINA LI-PING ET AL: "Regulation of type 2 innate lymphoid cell-dependent airway hyperreactivity by butyrate", JOURNAL OF ALLERGY AND CLINICAL IMMUNOLOGY, ELSEVIER, AMSTERDAM, NL, vol. 142, no. 6, 6 March 2018 (2018-03-06), pages 1867, XP085549828, ISSN: 0091-6749, DOI: 10.1016/J.JACI.2018.02.032
- TROMPETTE AURÉLIEN ET AL: "Dietary Fiber Confers Protection against Flu by Shaping Ly6c-Patrolling Monocyte Hematopoiesis and CD8+T Cell Metabolism", IMMUNITY, CELL PRESS, AMSTERDAM, NL, vol. 48, no. 5, 15 May 2018 (2018-05-15), pages 992, XP085397207, ISSN: 1074-7613, DOI: 10.1016/J.IMMUNI.2018.04.022
- WAT DENNIS: "The common cold: a review of the literature", EUROPEAN JOURNAL OF INTERNAL MEDICINE, 1 January 2004 (2004-01-01), Netherlands, pages 79 - 88, XP055817562, Retrieved from the Internet <URL:https://www.ncbi.nlm.nih.gov/pmc/articles/PMC7125703/pdf/main.pdf> [retrieved on 20210624], DOI: 10.1016/j.ejim.2004.01.006
- SCHÖGLER ALINE ET AL: "Vitamin D represses rhinovirus replication in cystic fibrosis cells by inducing LL-37", EUROPEAN RESPIRATORY JOURNAL, vol. 47, no. 2, 1 February 2016 (2016-02-01), GB, pages 520 - 530, XP055817617, ISSN: 0903-1936, Retrieved from the Internet <URL:https://erj.ersjournals.com/content/erj/47/2/520.full.pdf> DOI: 10.1183/13993003.00665-2015

## Description

The invention relates to a composition for use in the prevention and/ or treatment of an infection caused by Rhinovirus.

Rhinovirus is a primary cause of the common cold and is considered to be one of the most common viral infectious agents in humans. There are around 160 different serotypes of Rhinovirus that can infect humans that differ according to their protein composition. Acute Rhinovirus infection is a viral upper respiratory tract infection.

At present, there are no effective vaccines for the prevention of Rhinovirus infection in humans. Typically, children get infected with Rhinovirus around 8 to 12 times per year and adults get infected around 3 to 4 times per year. The approximate cost of Rhinovirus to the US economy is estimated to be around $40 billion every year.

Individuals suffering from the common cold or similar viral infections often take supportive treatments like pain killers or decongestants to relieve their symptoms. In addition, patients often purchase over the counter supplements such as those comprising Vitamin C, zinc and other substances that are thought to have an effect on enhancing the immune response. Examples of such supplements are the products 'Airborne' or 'Zicam'. Immune support products are part of a $500 million industry that is growing at around 5 to 6 percent per year.

Schögler et al (European Respiratory Journal, 47, No. 2, pages 520 to 530) refers to the repression of rhinovirus replication by Vitamin D in cystic fibrosis cells by inducing LL-37.

The present invention seeks to address the above problems.

According to a first aspect, there is provided a composition comprising sodium butyrate for use in the prevention and/ or treatment of a viral infection caused by Rhinovirus.

Preferably, there is provided a composition comprising sodium butyrate for use in the prevention and/ or treatment of acute Rhinovirus infection. Preferably, there is provided a composition comprising sodium butyrate for use in the prevention and/ or treatment of a viral upper respiratory tract infection. Preferably, the composition comprises sodium butyrate having the formula C4H7NaO2 (or C₄H₇NaO₂).

Advantageously, the use of the composition comprising sodium butyrate results in the reduction and/ or removal of Rhinovirus infection within an individual.

Advantageously, the use of the composition may have a prophylactic effect in relation to a viral infection caused by Rhinovirus.

Typically, the composition of the present invention acts through the stimulation of the immune response within an individual. Preferably, the composition stimulates the immune cells of an individual to release one or more substances that kill Rhinovirus and/ or inhibit its replication.

Advantageously, the use of the composition comprising sodium butyrate does not induce antimicrobial resistance within an individual since it works through stimulation of the innate immune response, rather than directly targeting the virus itself.

Advantageously, the composition of the present invention is easy to produce and cheap to purchase. Advantageously, the composition of the present invention does not produce any side effects within a patient when taken within recommended dosages.

In another embodiment, the composition further comprises Vitamin D. In one embodiment, the use of a composition comprising sodium butyrate and Vitamin D reduces and/ or removes the Rhinovirus infection within an individual. Typically, the combination of sodium butyrate and Vitamin D stimulates the immune response of a patient and reduces and/ or removes the Rhinovirus infection from the patient.

Typically, the composition comprises between 100 mg and 300 mg sodium butyrate. In one embodiment, the composition may comprise approximately 2000 mg sodium butyrate.

Typically, the composition further comprises between 400 IU and 4000 IU (i.e. between 10 µg and 100 µg) Vitamin D.

It is preferred that the composition is administered as a single dose per day.

In one embodiment, the composition is provided as a dietary supplement. Advantageously, the provision of the composition as a dietary supplement allows an individual to boost their own innate immune response to the virus. Advantageously, dietary supplementation with the composition comprising sodium butyrate and/ or Vitamin D enhances the host immune response to Rhinovirus.

Advantageously, sodium butyrate is a naturally occurring substance (a short chain fatty acid) that may be available to purchase from pharmacies and/ or chemists, or may be prescribed to patient as a supplement. Advantageously, sodium butyrate may be taken over the counter with no documented side effects within recommended doses.

In one embodiment, the composition may be administered by mouth. In a preferred embodiment, the composition may be administered in the form of a tablet or capsule. In another embodiment, the composition may be administered in the form of an inhaler, such as a nasal inhaler. Preferably, the composition is administered as a single dose, once per day.

In one embodiment, the composition may be coated with a material such as a lipid or sugar, or a combination thereof. Typically, the coating or target-guiding material may dissolve to allow efficient and sufficient delivery of the composition to cells, tissues or organs.

In one embodiment, the composition may be incorporated into a food or drink.

In one embodiment, the composition may comprise at least one acceptable carrier and may be carried within a vesicle, micelle, liposome, nanoparticle or other suitable vehicle.

According to a second aspect, there is provided a dietary supplement comprising a composition for use in accordance with the first aspect of the invention.

According to a third aspect, there is provided a tablet comprising a composition for use in accordance with the first aspect of the invention.

According to a fourth aspect, there is provided an inhaler comprising a composition for use in accordance with the first aspect of the invention.

In one embodiment, the inhaler is a nasal inhaler.

The invention will be further described by way of example and with reference to the following figures, in which:

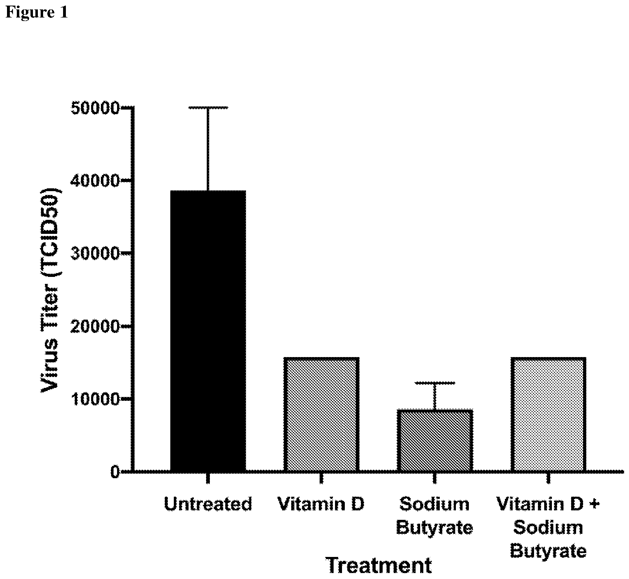

Figure 1 is a graph showing the effect on cells that have been infected with Rhinovirus following treatment with sodium butyrate, Vitamin D or a combination of sodium butyrate and Vitamin D.

Advantageously, the composition comprising sodium butyrate and/ or Vitamin D is for use in the reduction and/ or removal of Rhinovirus infection within an individual.

The composition in accordance with the present invention acts through the stimulation of the immune response within an individual, wherein the composition stimulates the immune cells of an individual to release one or more substances that kill/ inactivate Rhinovirus and/ or inhibit its replication.

In one embodiment, the composition is provided as a dietary supplement. Advantageously, dietary supplementation with sodium butyrate enhances the host's immune response to Rhinovirus. Preferably, the composition is provided in the form of a dietary supplement in pill form and marketed as an over the counter dietary supplement.

In another embodiment, the composition may be administered in the form of a tablet or capsule. In this embodiment, the composition may be coated with a material such as a lipid or sugar, or a combination thereof. Typically, the coating or target-guiding material dissolves to allow efficient and sufficient delivery of the conjugate to the cells, tissues or organs.

Pharmaceuticals, food supplements or dietary supplements often contain compounds and/ or other ingredients derived from a natural source or from a synthetic source. The natural or synthetic ingredient may be an active ingredient that provides a beneficial effect or may be an inactive ingredient.

Inactive ingredients, often termed excipients, are typically present within a pharmaceutical or a food or dietary supplement formulation. The choice of excipient or excipients in a formulation is dependent upon whether the active ingredients are to be delivered in a tablet form, a capsule form, in a liquid form or the manufacturing process.

The active or inactive ingredient may be a compound considered as "Generally Regarded As Safe" (GRAS) for human or animal consumption.

Excipients may be derived from a natural source or from a synthetic source. Excipients have a wide range of physical or chemical properties that are dependent on their structure. Excipient properties include but are not limited to anti-adhesion, binding, bulking, providing a protective or enteric coating, aiding dissolution or disintegration, providing a colouring, a flavouring or a sweetening effect, providing a flow or glidant effect, solvation, a sorbent effect or a lubricating effect that is beneficial in formulation, manufacturability or the delivery of the active ingredients.

An example of the composition according to the invention is described below and contains both active ingredients and excipient ingredients that are common within pharmaceuticals and food or dietary supplements:
Active Ingredients:

| | |
|---|---|
| Vitamin D (CAS No: 69-97-0) | 25 micrograms (1000 IU or 25ug) |
| Sodium Butyrate (CAS No: 156-54-7) | 300 milligrams (mg) |

Excipients/ Inactive Ingredients:

| | |
|---|---|
| Microcrystalline Cellulose (CAS No: 9004-34-6) | 350 milligrams (mg) |
| Calcium Diphosphate (CAS no: 7758-87-4) | 300 milligrams (mg) |
| Silica SiO2 (CAS No: 7631-86-9) | 20 milligrams (mg) |
| Magnesium Stearate (CAS No: 557-04-0) | 30 milligrams (mg) |

The above example formulation is for a single, solid dose to be given orally.

Those skilled in the art will understand the benefit in varying the ratio of the ingredients within the above example formulation to enable volume manufacturing of tablets or capsules for human or animal consumption.

In another embodiment, the composition may be prepared comprising sodium butyrate as an active ingredient and the excipients/ inactive ingredients microcrystalline cellulose, calcium diphosphate, silica SiO2 and magnesium stearate in the amounts as mentioned above.

With reference to Figure 1, an experiment was carried out to treat leukocytes (the human monocytic THP-1 macrophage cell line differentiated into macrophages) with sodium butyrate, Vitamin D, or with a combination of sodium butyrate and Vitamin D.

The leukocyte cells were incubated for a period of time to allow them to release proteins into the surrounding cell culture media (referred to as `conditioned media'). The conditioned media was then incubated with HRV1B Rhinovirus. The leukocyte cells were incubated with a solution comprising 0.5 mM sodium butyrate, a solution comprising 0.5 mM Vitamin D, and with a solution comprising both 0.5 mM sodium butyrate and 0.5 mM Vitamin D.

The results shown in Figure 1 demonstrate that incubation with sodium butyrate resulted in a 75-80% reduction in measurable live virus, when compared with the control (treated with distilled water). In addition, incubation with Vitamin D alone, or with sodium butyrate and Vitamin D together resulted in a significant reduction in measureable live virus when compared with the control.

The data shown is an average of n=3 independent biological replicates (the titration is an average often fold reductions quantified over the three replicates).

Sodium butyrate and Vitamin D have effects on different intracellular mediators and/ or signalling molecules in order to provide an effective response to viral infection. Thus, the provision of a composition comprising both sodium butyrate and Vitamin D would be advantageous since some individuals may be more responsive, in the context of their innate immunity, to either sodium butyrate or Vitamin D. Thus, administering a composition comprising both sodium butyrate and Vitamin D would provide an effective reduction in viral infection in a broader range of people.

Butyrate has been shown to increase cathelicidin production (Schauber et al, Gut 2003, 52(5), 735-741 and Hase et al, Infect Immun 2002, 70 (2), 953-963).

With reference to the present invention, it is understood that the sodium butyrate reduces the level of Rhinovirus titre through the stimulation of cathelicidins.

In addition, downregulation of inflammation in the context of viral infection can be an additional beneficial outcome since in Rhinovirus infection, much of the symptomology can be attributed to virus induced inflammation (Greenberg, Arch Int Med, 2003 163(3), 278-284). It has been shown in mouse models of influenza infection that exogenous LL-37 (a human member of the cathelicidin family) can decrease inflammation (Barlow et al, PLos One, 2011, 6(10)), thus resulting in enhanced survival rates. It has also been demonstrated that exogenous LL-37 can reduce Rhinovirus induced inflammatory cytokine release in cell models (Casanova et al, Front Immunol 2020, 11 (85).

With reference to the present invention, it is understood that sodium butyrate can reduce inflammation caused by viral infection through a reduction in NFkappa B signalling which would be of benefit for relief of the symptoms of Rhinovirus infection.

## Claims

1. A composition comprising sodium butyrate for use in the prevention and/ or treatment of a viral infection caused by Rhinovirus.

2. A composition for use according to claim 1, wherein the composition stimulates the immune cells of an individual to release substances that kill Rhinovirus and/ or inhibit its replication.

3. A composition for use according to any preceding claim, wherein the composition further comprises Vitamin D.

4. A composition for use according to any preceding claim, wherein the composition is administered by mouth.

5. A composition for use according to claim 4, wherein the composition is administered in the form of a tablet or capsule.

6. A composition for use according to any one of claims 1 to 3, wherein the composition is administered in the form of an inhaler.

7. A composition for use according to any one of the preceding claims, wherein the composition comprises at least one acceptable carrier and may be carried within a vesicle, micelle, liposome, nanoparticle or other suitable vehicle.

8. A composition for use according to any preceding claim, wherein the composition is provided as a dietary supplement.

9. A composition for use according to any preceding claim, wherein the composition is incorporated into a food or drink.

10. A dietary supplement comprising a composition comprising sodium butyrate for use in the prevention and/ or treatment of a viral infection caused by Rhinovirus.

11. A dietary supplement for use according to claim 10, wherein the composition further comprises Vitamin D.

12. A tablet comprising a composition comprising sodium butyrate for use in the prevention and/ or treatment of a viral infection caused by Rhinovirus.

13. A tablet for use according to claim 12, wherein the composition further comprises Vitamin D.

14. An inhaler comprising a composition comprising sodium butyrate for use in the prevention and/ or treatment of a viral infection caused by Rhinovirus.

15. An inhaler for use according to claim 14, wherein the composition further comprises Vitamin D.

## Patentansprüche

1. Zusammensetzung, die Natriumbutyrat umfasst, für die Verwendung bei der Prävention und/oder Behandlung einer Virusinfektion, die durch Rhinovirus verursacht wird.

2. Zusammensetzung für die Verwendung nach Anspruch 1, wobei die Zusammensetzung die Immunzellen eines Individuums stimuliert, um Substanzen freizusetzen, die Rhinovirus abtöten und/oder dessen Replikation hemmen.

3. Zusammensetzung für die Verwendung nach einem vorstehenden Anspruch, wobei die Zusammensetzung weiter Vitamin D umfasst.

4. Zusammensetzung für die Verwendung nach einem vorstehenden Anspruch, wobei die Zusammensetzung über den Mund verabreicht wird.

5. Zusammensetzung für die Verwendung nach Anspruch 4, wobei die Zusammensetzung in Form einer Tablette oder Kapsel verabreicht wird.

6. Zusammensetzung für die Verwendung nach einem der Ansprüche 1 bis 3, wobei die Zusammensetzung in Form eines Inhalators verabreicht wird.

7. Zusammensetzung für die Verwendung nach einem der vorstehenden Ansprüche, wobei die Zusammensetzung mindestens einen verträglichen Träger umfasst und innerhalb eines Vesikels, einer Mizelle, eines Liposoms, Nanopartikels oder anderen geeigneten Vehikels getragen werden kann.

8. Zusammensetzung für die Verwendung nach einem vorstehenden Anspruch, wobei die Zusammensetzung als eine Nahrungsergänzung bereitgestellt wird.

9. Zusammensetzung für die Verwendung nach einem vorstehenden Anspruch, wobei die Zusammensetzung in ein Nahrungsmittel oder Getränke eingearbeitet ist.

10. Nahrungsergänzung, die eine Zusammensetzung umfasst, die Natriumbutyrat umfasst, für die Verwendung bei der Prävention und/oder Behandlung einer Virusinfektion, die durch Rhinovirus verursacht wird.

11. Nahrungsergänzung für die Verwendung nach Anspruch 10, wobei die Zusammensetzung weiter Vitamin D umfasst.

12. Tablette, die eine Zusammensetzung umfasst, die Natriumbutyrat umfasst, für die Verwendung bei der Prävention und/oder Behandlung einer Virusinfektion, die durch Rhinovirus verursacht wird.

13. Tablette für die Verwendung nach Anspruch 12, wobei die Zusammensetzung weiter Vitamin D umfasst.

14. Inhalator, der eine Zusammensetzung umfasst, die Natriumbutyrat umfasst, für die Verwendung bei der Prävention und/oder Behandlung einer Virusinfektion, die durch Rhinovirus verursacht wird.

15. Inhalator für die Verwendung nach Anspruch 14, wobei die Zusammensetzung weiter Vitamin D umfasst.

## Revendications

1. Composition comprenant du butyrate de sodium, pour une utilisation dans la prévention et/ou le traitement d'une infection virale causée par un Rhinovirus.

2. Composition pour une utilisation selon la revendication 1, la composition stimulant les cellules immunitaires d'un individu afin de libérer des substances qui tuent le Rhinovirus et/ou inhibent sa réplication.

3. Composition pour une utilisation selon l'une quelconque des revendications précédentes, la composition comprenant en outre de la vitamine D.

4. Composition pour une utilisation selon l'une quelconque des revendications précédentes, la composition étant administrée par voie orale.

5. Composition pour une utilisation selon la revendication 4, la composition étant administrée sous la forme d'un comprimé ou d'une capsule.

6. Composition pour une utilisation selon l'une quelconque des revendications 1 à 3, la composition étant administrée sous la forme d'un inhalateur.

7. Composition pour une utilisation selon l'une quelconque des revendications précédentes, la composition comprenant au moins un véhicule acceptable et pouvant être transportée au sein d'une vésicule, d'une micelle, d'un liposome, d'une nanoparticule ou d'un autre véhicule convenable.

8. Composition pour une utilisation selon l'une quelconque des revendications précédentes, la composition étant fournie sous forme de complément alimentaire.

9. Composition pour une utilisation selon l'une quelconque des revendications précédentes, la composition étant incorporée dans un aliment ou une boisson.

10. Complément alimentaire comprenant une composition comprenant du butyrate de sodium, pour une utilisation dans la prévention et/ou le traitement d'une infection virale causée par un Rhinovirus.

11. Complément alimentaire pour une utilisation selon la revendication 10, dans lequel la composition comprend en outre de la vitamine D.

12. Comprimé comprenant une composition comprenant du butyrate de sodium, pour une utilisation dans la prévention et/ou le traitement d'une infection virale causée par un Rhinovirus.

13. Comprimé pour une utilisation selon la revendication 12, dans lequel la composition comprend en outre de la vitamine D.

14. Inhalateur comprenant une composition comprenant du butyrate de sodium, pour une utilisation dans la prévention et/ou le traitement d'une infection virale causée par un Rhinovirus.

15. Inhalateur pour une utilisation selon la revendication 14, dans lequel la composition comprend en outre de la vitamine D.
